(19) Europäisches Patentamt
European Patent Office
Office européen des brevets

(11) EP 0 685 489 B1

(12) **EUROPEAN PATENT SPECIFICATION**

(45) Date of publication and mention of the grant of the patent:
**19.12.2001 Bulletin 2001/51**

(51) Int Cl.$^7$: **C07K 5/08**

(21) Application number: **95201960.2**

(22) Date of filing: **26.09.1991**

(54) **Tripeptide antithrombotic agents**

Tripeptidisches antithrombotisches Mittel

Tripeptide, qui est un agent antithrombotique

(84) Designated Contracting States:
**AT BE CH DE DK ES FR GB GR IT LI LU NL SE**

(30) Priority: **28.09.1990 US 589553**
**06.09.1991 US 756091**

(43) Date of publication of application:
**06.12.1995 Bulletin 1995/49**

(62) Document number(s) of the earlier application(s) in accordance with Art. 76 EPC:
**91308785.4 / 0 479 489**

(73) Proprietor: **ELI LILLY AND COMPANY**
**Indianapolis, Indiana 46285 (US)**

(72) Inventors:
- **Gesellchen, Paul David**
**Indianapolis, Indiana 46240 (US)**
- **Shuman, Robert Theodore**
**Greenwood, Indiana 46142 (US)**

(74) Representative: **Pritchard, Judith et al**
**Eli Lilly and Company Limited Lilly Research Centre Erl Wood Manor**
**Windlesham, Surrey GU20 6PH (GB)**

(56) References cited:
**EP-A- 0 185 390** **EP-A- 0 335 662**
**EP-A- 0 367 713** **DE-A- 3 827 415**
**US-A- 4 346 078**

- **J MED CHEM 1990, pages 1729 - 1735**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 685 489 B1

## Description

This invention relates to thrombin inhibitors which are useful anticoagulants in humans and animals. In particular it relates to derivatives of the dipeptide L-Proline-L-Arginine aldehyde having high antithrombotic activity.

Thrombin inhibition is currently achieved by the administration of heparins and coumarins. The mechanism by which these agents act has been much studied. Heparins are only administerable parenterally and levels must be carefully monitored. Coumarins act by blocking or inhibiting the formation of prothrombin and require some time to achieve maximum effectiveness.

Although both the heparins and the coumarins are effective anticoagulants there exists a need for antithrombin agents which act quickly to prevent clot formation and which do not interfere with plasmin action in dissolving existing clots.

The thrombin inhibiting compounds provided by this invention are represented by the following formula 1.

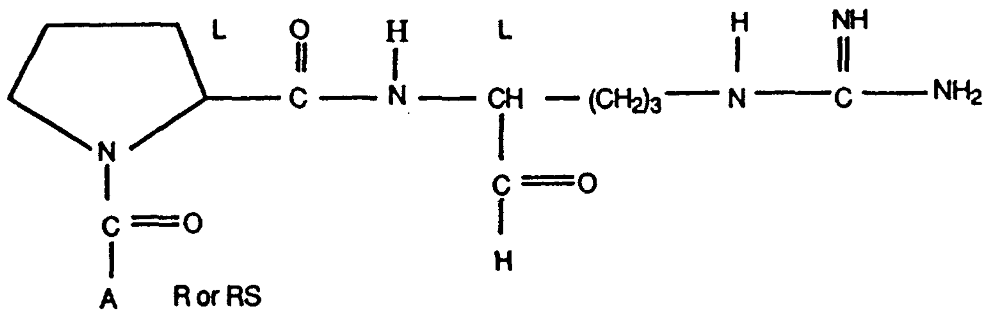

1

wherein A is

1-aminocyclohexyl or 1-aminocyclopentyl wherein the amino group is an $-N(R_2)(R_3)$ wherein $R_2$ and $R_3$ independently are hydrogen or lower alkyl or $R_2$ is hydrogen and $R_3$ is acetyl, haloacetyl or an oxycarbonyl group of the formula

$$R_4\text{-O-C(O)-}$$

wherein $R_4$ is $C_1$-$C_6$ alkyl, $C_2$-$C_6$ alkenyl, $C_3$-$C_7$ cycloalkyl, benzyl, nitrobenzyl, diphenylmethyl, or a phenyl group of the formula

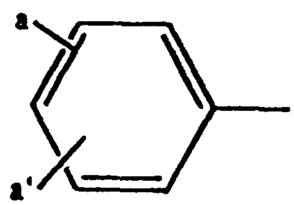

wherein a and a' independently are hydrogen, lower alkyl, lower alkoxy, halogen, trifluoromethyl, hydroxy, hydroxymethyl, amino, or aminomethyl; and the pharmaceutically acceptable non-toxic salts thereof.

The peptides represented by the formula 1 are useful antithrombotic agents and can be used as adjuncts to tissue plasminogen activator (tPA), streptokinase or urokinase therapy.

The compounds are prepared by conventional coupling methods. For example, Boc-D-Phg is coupled with an ester of L-proline to form Boc-D-Phg-Pro ester. The ester group is removed and the Boc-D-Phg-Pro is coupled with the lactam form of L-arginine to provide Boc-D-Phg-Pro-Arg lactam in amino protected form. The Arg lactam ring is opened by reduction and the arginine amino protecting group removed to provide Boc-D-Phg-Pro-Arg aldehyde. The peptides are converted to suitable salt forms such as the acetates and sulfates.

The invention also provides a method for preventing the formation of clots in man and animals and pharmaceutical formulations useful in the method.

As shown in formula 1, the asymmetric center of the A(C=0) moiety is R or RS while that of the proline and arginine aldehyde moieties is L.

The terms used in formula 1 are defined herein as follows:

Lower alkyl refers to the straight and branched chain $C_1$-$C_4$ alkyl groups such as methyl, ethyl, n-propyl, isopropyl, n-butyl and the like.

Lower alkoxy refers to $C_1$-$C_4$ alkoxy group such as methoxy, ethoxy, n-propoxy, isopropoxy, n-butoxy, t-butoxy and the like.

Halogen refers to fluoro, chloro, bromo or iodo.

Mono- or di-(lower alkyl)amino refers to such groups as methylamino, ethylamino, dimethylamino, methylethylamino, diethylamino, n-butylamino, n-propylamino and the like.

The term "$C_1$-$C_6$ alkyl" refers to the straight and branched alkyl groups such as the $C_1$-$C_4$ alkyl groups defined above and, in addition, n-pentyl, isopentyl, n-hexyl, the isomeric hexyl groups, and the like. "$C_2$-$C_6$ alkenyl" refers to the olefinic groups such as vinyl, allyl, butenyl, isomeric pentenyl and hexenyl groups. "$C_3$-$C_7$ Cycloalkyl" refers to the cyclic hydrocarbons having from three to 7 ring carbon atoms such as cyclopropyl, cyclobutyl, cyclopentyl, cyclohexyl and cycloheptyl.

As defined in formula 1, $R_4$ can be a phenyl group which may be mono- or di-substituted. Examples of such phenyl groups are phenyl (a and a' = H), 4-methylphenyl, 3-ethylphenyl, 4-methoxyphenyl, 3-methoxyphenyl, 3-ethoxyphenyl, 2-methoxyphenyl, 3-isopropoxyphenyl, 4-hydroxyphenyl, 4-chlorophenyl, 3-chlorophenyl, 2-fluorophenyl, 3-fluorophenyl, 3-bromophenyl, 4-fluorophenyl, 3-trifluoromethylphenyl, 4-trifluoromethylphenyl, 4-hydroxymethylphenyl, 2-hydroxymethylphenyl, 3-aminophenyl, 4-aminophenyl, 3-amino-4-chlorophenyl, 3,4-dichlorophenyl, 3-hydroxy-4-fluorophenyl, 3-hydroxy-4-methylphenyl, 3-methoxy-4-hydroxyphenyl, 3-chloro-4-ethoxyphenyl, and like mono- or di-substituted phenyl groups.

Examples of groups represented in the formula 1 by the amino group -N($R_2$)($R_3$) are amino ($R_2$ = $R_3$ = H), methylamino, ethylamino, isopropylamino, dimethylamino, and like amino groups; and when $R_2$ is hydrogen and $R_3$ is an oxycarbonyl group $R_4$-O-C(O)-, examples of such groups, are the $C_1$-$C_6$ alkoxycarbonylamino groups such as methoxycarbonylamino, ethoxycarbonylamino, t-butoxycarbonylamino, isoamyloxycarbonylamino and the like; the $C_2$-$C_6$ alkenyloxycarbonylamino groups such as vinyloxycarbonylamino, allyloxycarbonylamino, 2-butenyloxycarbonylamino, and the like; $C_3$-$C_7$ cycloalkoxycarbonylamino groups such as cyclopropyloxycarbonylamino, cyclopentyloxycarbonylamino, cyclohexyloxycarbonylamino, and the like. Oxycarboxylamino groups represented further include for example, benzyloxycarbonylamino, 4-nitrobenzyloxycarbonylamino, diphenylmethoxycarbonylamino, phenyloxycarbonylamino, or a substituted phenyloxycarbonylamino group wherein the substituted phenyl moiety is as defined hereinabove, and the like.

The peptide compounds represented by the formula 1 wherein A is an achiral 1-aminocyclopentyl or 1-amino cyclohexyl group are depicted by the following structural formula.

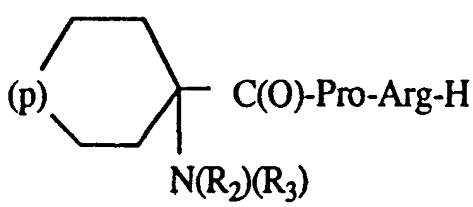

wherein p is a carbon to carbon bond or $-CH_2-$; and $R_2$ and $R_3$ have the same meanings as defined hereinabove. Examples of such tripeptides are N-(1-aminocyclohexanoyl)-Pro-Arg-H, N-(1-aminocyclopentanoyl)-Pro-Arg-H; N-(1-methylaminocyclohexanoyl)-Pro-Arg-H, N-(1-t-butyloxycarbonylaminocyclohexanoyl)-Pro-Arg-H, and the like.

Pharmaceutically acceptable salts of peptides of the invention include the acid addition salts formed with inorganic acids and carboxylic acids. Examples of inorganic acids forming salts are the hydrohalic acids hydrochloric and hydrobromic; phosphoric acid and sulfuric acid. Carboxylic acid salts are formed with acids such as acetic, propionic, malonic, maleic, citric, succinic, malic, benzoic, fumaric, and like carboxylic acids. The acid addition salts are prepared in a conventional manner e.g. by neutralizing the free base form of the compound 1 with the acid. Preferred acid addition salts are sulfate and hydrochloride salts.

The compounds represented by the formula 1 are prepared by known methods of peptide coupling. According to one such method the acid A-COOH, wherein A has the same meanings as defined for formula 1, is coupled with a carboxy protected proline to form the dipeptide (when A is an amino acid) or an N-acylproline ester (when A is other than an amino acid). The carboxy protecting ester group of the proline moiety of the product is removed and the free acid form of the dipeptide is coupled with the lactam form of arginine. The above reaction sequence is illustrated by

the following scheme

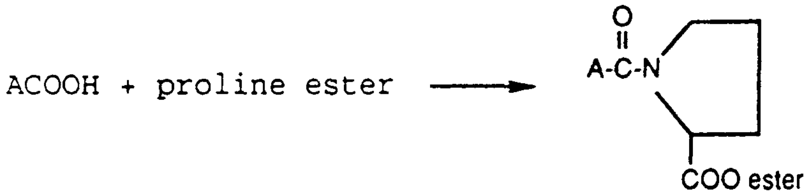


(a)

(a) —deesterify→ A-(C=O)-Pro-OH (b)

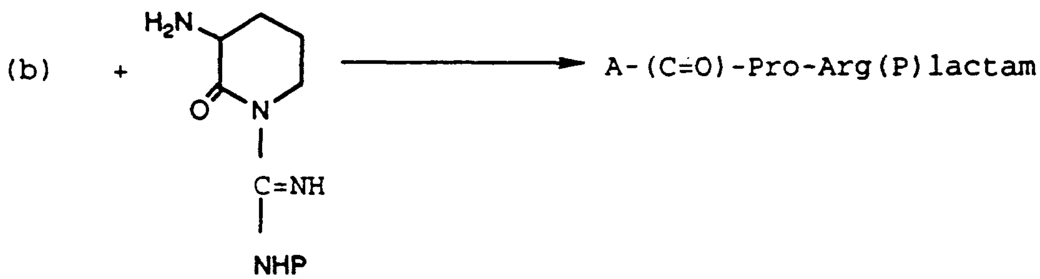


(c)

wherein P represents an amino protecting group.

The coupled Arg(P) lactam product (c) is reduced with lithium aluminum hydride in an inert solvent to cleave the lactam ring and provide the tripeptide in the arginine aldehyde form represented by the formula

A(C=O)-Pro-Arg(P)-H

wherein Arg(P)-H represents amino protected arginine aldehyde.

The lactam form of arginine is obtained by intramolecular coupling of amino protected arginine [Arg-OH]. For example, Boc-Arg(Cbz)OH represented by the formula

```
Boc-NH-CH-(CH2)3-NH-C(=NH)-NHCbz
        |
       COOH
```

is first converted to an active ester form, such as an active mixed anhydride, with a chloroformate ester, e.g. ethyl chloroformate to isobutyl chloroformate. The ester formation is carried out in the presence of a tertiary amine such as N-methylmorpholine. Addition of a stronger tertiary amine base such as triethylamine effects the internal acylation to provide the lactam form of the di-amino protected arginine as shown below.

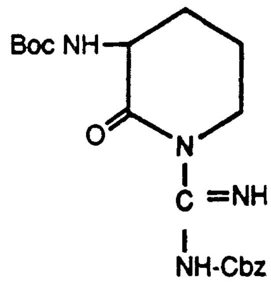

Prior to use in the coupling with the A(C=O)-Pro-OH as shown in the above scheme, the Boc protecting group is selectively removed with trifluoroacetic acid to provide the requisite free amino group.

The coupling of an ACOOH compound with a proline ester, when A is an amino acid residue, is carried out by first protecting the amino group of the amino acid. Conventional amino protecting groups commonly used for temporary protection or blocking of the amino group are employed. Examples of such protecting groups include the alkoxy, alkenyloxy, cycloalkoxy and aryloxycarbonyl groups such as ethoxycarbonyl, t-butyloxycarbonyl, cyclohexyloxycarbonyl, adamantyloxycarbonyl, trichloroethoxycarbonyl, benzyloxycarbonyl, diphenylmethoxycarbonyl, and like groups. The ester group employed to protect the carboxy group of proline during the coupling reaction can be any of the commonly used readily removable ester groups such as t-butyl, benzyl, p-nitrobenzyl, p-methoxybenzyl, diphenylmethyl, trichloroethyl, phenacyl, or trialkylsilyl esters. In carrying out the coupling reaction one employs an ester group for proline which is removable by conditions under which the amino protecting group remains intact. The amino protecting group of the acylating acid ACOOH thus remains in place for protection of the amino group during the subsequent coupling with the arginine lactam compound to form c.

For example, N-methyl-D-phenylglycyl-L-prolyl-L-arginine aldehyde is prepared by reductive alkylation as follows. Cbz protected D-phenylglycine is coupled in DMF with L-proline t-butyl ester using dicyclohexylcarbodiimide (DCC) and hydroxybenzotriazole (HOBt) to form the dipeptide Cbz-D-phenylglycyl-L-proline t-butyl ester. The peptide is hydrogenated in ethyl alcohol over palladium on carbon catalyst to remove the Cbz protecting group, formaldehyde is added to the reduction mixture and the hydrogenation is continued to form N-methyl-D-phenylglycyl-L-proline t-butyl ester. The N-methyl secondary amino group of the phenylglycyl moiety is protected with the Cbz group by reacting the dipeptide t-butyl ester with benzyl chloroformate in THF containing N-methylmorpholine to form N-Cbz-N-methyl-D-phenylglycyl-L-proline t-butyl ester. The t-butyl ester group is removed at room temperature in trifluoroacetic acid containing anisole to provide N-Cbz-N-methyl-D-phenylglycyl-L-proline. The latter dipeptide is then coupled to the Cbz protected Arg lactam and the lactam ring reductively opened to the Arg aldehyde as described above. Both of the Cbz protecting groups of the tripeptide are removed by hydrogenation over Pd/C catalyst to provide N-methyl-D-phenylglycyl-L-prolyl-L-arginine aldehyde.

The coupling reactions described above are carried out in the cold preferably at a temperature between about -20° C and about 15° C. The coupling reactions are carried out in an inert organic solvent such as dimethylformamide, dimethylacetamide, tetrahydrofuran, methylene chloride, chloroform, and like common solvents. Generally anhydrous conditions are used when, in the coupling reaction, an active ester of the acylating acid is used.

The compounds of the invention are isolated best in the form of acid addition salts. Salts of the compounds of formula 1 formed with acids such as those mentioned hereinabove useful as pharmaceutically acceptable salts for administration of the antithrombotic agents and for preparation of formulations of these agents. Other acid addition salts may be prepared and used in the isolation and purification of the peptides. For example, the salts formed with the sulfonic acids such as methanesulfonic acid, n-butanesulfonic acid, p-toluenesulfonic acid and naphthalene sulfonic acid may be so used.

The preferred method for isolating and purifying the compounds represented by the formula 1 while at the same time preparing a desired stable salt form is as follows. Stable salts of inorganic acids such as the sulfate and hydrochloride salts are provided by preparative purification over $C_{18}$ reversed-phase chromatography. The aqueous phase comprises sulfuric acid or hydrochloric acid at a concentration between about 0.01% and about 0.05% and acetonitrile, THF, methanol or other suitable solvent serves as the organic component. The pH of the acidic eluant is adjusted to between about pH 4 and about pH 6, the exact pH being a function of the particular peptide, with a basic resin e.g. Bio-Rad AG-1X8 resin in the hydroxyl form. After pH adjustment the solution of the tripeptide salt e.g. sulfate or hydrochloride, is lyophilized to provide the purified salt dry powder form. In an example of the process crude D-Phg-L-Pro-L-Arg-H sulfate, contaminated with the epimeric D-Arg-H sulfate is dissolved in ca 0.01% sulfuric acid and the solution is loaded on a Vydac $C_{18}$ RP-HPLC column. A gradient of 2-10% acetonitrile in 0.01% $H_2SO_4$ was used to elute the column over 10 hours. Multiple fractions are collected and those containing the desired product as determined

by analytical RP-HPLC are pooled. The pH of the pooled fractions is adjusted to about pH 4.0 to about 4.5 with the Bio-Rad AG-1X8 resin in the hydroxyl cycle. After filtering the solution is lyophilized to provide pure D-Phg-L-Pro-L-Arg-H sulfate.

The compounds provided by the invention (formula 1) inhibit the action of thrombin in man and animals. The inhibition of thrombin is demonstrated by in vitro inhibition of amidase activity of thrombin. The following Table 1 lists the apparent equilibrium constant (Kass) for interaction between the test compound (inhibitor) and thrombin. The data in the table were obtained in an assay in which thrombin hydrolyzes the chromogenic substrate, N-benzoyl-D-phenylalanyl-L-valyl-L-arginyl-p-nitroanilide.

The assay was carried out in 50 µl buffer (0.03M Tris, 0.15M NaCl, pH 7.4) with 25 µl of thrombin solution (0.21 mg/ml of thrombostat powder in 0.06 M Tris, 0.3M NaCl, pH 7.4) and 150 µl of an aqueous solution of the chromogenic substrate at a concentration of 0.25 mg/ml. Solutions of test compound (25 µl) at various concentrations were added. Rates of hydrolysis of the substrate were measured by monitoring the reactions at 405 nm for the release of p-nitroaniline. Standard curves were constructed by plotting free thrombin concentration against hydrolysis rate. The hydrolysis rates observed with test compounds are then converted to "free thrombin" values in the respective assays by use of the standard curves. The bound thrombin (bound to test compound) was calculated by subtracting the amount of free thrombin observed in each assay from the known initial amount of thrombin used in the assay. The amount of free inhibitor in each assay was calculated by subtracting the number of moles of bound thrombin from the number of moles of added inhibitor (test compound).

The Kass value is the hypothetical equilibrium constant for the reaction between thrombin and the test compouhnd (I).

$$\text{Thrombin} + \text{I} \rightleftharpoons \text{Thrombin - I}$$

$$\text{Kass} = \frac{[\text{Thrombin I}]}{[\,(\text{Thrombin}) \times (\text{I})\,]}$$

Kass was calculated for a range of concentrations of test compounds and the mean value calculated in units of liter per mole.

The anticoagulant activity of the compounds of the invention has been determined in standard tests used to determine prothrombin time, thrombin time and activated partial thromboplastin time (APTT). The concentrations of the test compounds (ng/ml) required to prolong coagulation by 2-fold in the three tests were measured. The thrombin time evaluation was carried out in plasma and separately determined in a buffer system at pH 7.5.

**TABLE**

**Anticoagulant Activity of Thrombin Inhibitors**

| Test Inhibitor[1] | Plasma Coagulation Assay[2] | | | Buffer (pH 7.5)[2] |
|---|---|---|---|---|
| A(C=O) | Prothrombin Time | APTT[3] | Thrombin Time | Thrombin Time |
| D-Phenylglycyl | 91 | 1139 | 91 | NA[4] |
| N-Boc-D-Phenylglycyl[5] | 667 | 1169 | 110 | 118 |
| N-Boc-D-(4-methoxy)phenylglycyl | NA | 640 | 80 | NA |
| N-Boc-D-(4-hydroxy)phenylglycyl | 1636 | 1091 | 182 | NA |
| N-Boc-DL-1-naphthylglycyl | 1136 | 2272 | 320 | 112 |
| N-Boc-D-1-naphthylglycyl | 1225 | 3100 | 230 | 105 |
| N-Boc-D-2-naphthylglycyl | 1200 | 1200 | 230 | 142 |
| N-Acetyl-D-(4-methoxy)phenylglycyl | 900 | 2600 | 160 | NA |
| N-Boc-D-(6-methoxy)-2-naphthylglycyl | 35489 | 2239 | 460 | 466 |
| N-Boc-D-Cyclohexylglycyl | 522 | 748 | 79 | 60 |
| N-Boc-N-Methyl-D-Phenylglycyl | 300 | 4545 | 300 | NA |
| N-Methyl-D-Phenylglycyl | 899 | >909 | 92 | 67 |
| (R)-α-Ethylphenylacetyl | 700 | 2275 | 115 | NA |
| (R)-α-Methoxyphenylacetyl | >909 | >909 | 139 | 173 |

1/ A(C=O) for formula 1
2/ Concentration (ng/ml) of test compounds required to prolong coagulation by 2-fold
3/ APTT = Activated Partial Thromboplastin Time
4/ NA = Not available
5/ The values are averages of 8 assays

The data were obtained using a CoaScreener instrument from Tecan, Inc. in the assay protocols listed below.

| | |
|---|---|
| Prothrombin Time | 50 µl plasma<br>50 µl saline<br>7 µl test solution<br>50 µl thromboplastin (Dade) |

| | |
|---|---|
| Thrombin Time | 50 µl plasma<br>50 µl saline<br>7 µl test compound<br>50 µl bovine thrombin (2 NIH unit/ml) |

In the Thrombin Time determined in pH 7.4 buffer fibrinogen was used instead of plasma.

| | |
|---|---|
| APTT | 50 µl plasma<br>50 µl Actin (Dade)<br>7 µl test solution<br>50 µl $CaCl_2$ (0.01M). |

The antithrombotic activity of representative compounds of the invention was determined in in vivo tests carried out in the rat. The test employed induced arterial thrombosis in the carotid artery of the rat and measured the infusion dose of test compound required to maintain blood flow for fifty minutes past the time of occlusion. The test was performed as follows.

Arterial thrombosis was induced in the rat by injury of the carotid artery. Topical application of a ferric chloride solution was used to injure the vessel. Male Sprague-Dawley rats (375-450 g) were anesthetized with xylazine (20 mg/kg, s.c.) followed by ketamine HCl (100 mg/kg, s.c.). Animals were laid on a water blanket in which the circulated water was maintained at 37° C. The carotid artery was approached through a midline cervical incision. Careful blunt dissection was used to expose and isolate the vessel from the carotid sheath. A silk suture was pulled underneath the artery to lift the vessel to provide clearance to insert a thermocouple underneath it. Vessel temperature changes were monitored on a strip chart recorder that had an ink-writing timer. Small forceps were used to dip discs (3 mm dia) of Whatman No. 1 filter paper into a $FeCl_3$ solution (35%). The discs were cut to equal size using sharpened stainless steel tubing (3 mm i.d.) chucked in a drill press. A saturated disc was placed on each carotid artery above the thermocouple. The time between $FeCl_3$ application and the time at which temperature decreased abruptly was recorded as time to occlusion (TTO) of the vessel. The average time required for both vessels to occlude was used to represent TTO for each animal.

Test compounds were dissolved in isotonic saline. A syringe pump was used to infuse drug solutions starting 15 min before $FeCl_3$ application and continuing for 60 min after $FeCl_3$ application. Dose-response curves were plotted to determine the relationship between the $\log_{10}$ of the infused doses and the TTO of the injured arteries. A comparative index of antithrombotic activity was determined from the curves by calculating the infusion dose required to maintain blood flow for 50 min (ED 50 min).

The association between vessel occlusion and the abrupt temperature decrease was established by simultaneously recording the temperature and blood flow on the same recorder. A pulsed Doppler flow probe was placed around the carotid artery proximal to the thermocouple. The probe recorded changes in flow velocity; therefore, it was installed at a point where thrombosis did not occur and the internal diameter of the vessel remained constant due to distention with fluid blood. Baseline temperature and flow velocity (determined with a Directional Pulsed Doppler Flowmeter, Model 545-C, University of Iowa Bioengineering) were recorded before application of 35% ferric chloride. Results were reported as percent change from initial baseline values (6 min before occlusion). The time at which vessel temperature decreased rapidly was arbitrarily established as zero and pre- and post-occlusion temperature and flow values were referenced from that point.

The compounds of the invention inhibit clot formation without appreciable interference with the bodies natural clot lysing ability e.g.the compounds have a low inhibitory effect on fibrinolysis.

The invention in one of its aspects provides a method for inhibiting the formation of blood clots in man and animals which comprises administering to said man or animal an effective clot inhibiting non-toxic dose of a compound represented by the formula 1. The anti-coagulant compound is administered orally, parenterally e.g. by intravenous infusion (iv), intramuscular injection (im) or subcutaneously (sc). Preferably administration is carried out by iv infusion.

An effective clot inhibiting dose is between about 5 mg and about 1000 mg. The dose regime may vary e.g. for prophylactic use a single daily dose may be administered or multiple doses such as 3 or 5 times daily may be appropriate. In critical care situations a compound of the invention is administered by iv infusion at a rate between about 1 mg/kg/h and about 50 mg/kg/h and preferably between about 2.5 mg/kg/h and about 25 mg/kg/h.

The method of this invention also is practiced in conjunction with a clot lysing agent e.g. tissue plasminogen activator (tPA), modified tPA, streptokinase or urokinase. In cases when clot formation has occurred and an artery or vein is blocked, either partially or totally, a clot lysing agent is usually employed. A compound of the invention can be administered along with the lysing agent or subsequent to its use to prevent the reoccurrence of clot formation.

In carrying out the method the use of a preferred compound of the invention is desirable. For example use is made of a preferred compound such as described hereinabove.

The invention also provides pharmaceutical formulations for use in the above described therapeutic method. Pharmaceutical formulations of the invention comprise an effective clot inhibitory amount of a compound represented by the formula 1 and a pharmaceutically acceptable carrier. For oral administration the antithrombotic compound is formulated in gelatin capsules or tablets which may contain excipients such as binders, lubricants, disintegration agents and the like. For parenteral administration the antithrombotic is formulated in a pharmaceutically acceptable diluent e. g. physiological saline (0.9%), 5% dextrose, Ringer's solution and the like.

The antithrombotic compound of the invention can be formulated in unit dosage formulations comprising a dose between about 1 mg and about 1000 mg. Preferably the compound is in the form of a pharmaceutically acceptable salt such as for example the sulfate salt, acetate salt or a phosphate salt.

## Claims

**Claims for the following Contracting States : AT, BE, CH, LI, DE, DK, FR, GB, IT, LU, NL, SE**

1. A compound of the formula

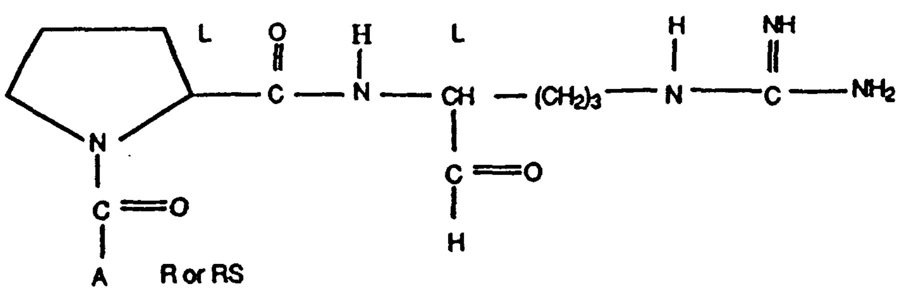

1

wherein A is 1-aminocyclohexyl or 1-aminocyclopentyl wherein the amino group is an -N($R_2$)($R_3$) group wherein $R_2$ and $R_3$ independently are hydrogen or lower alkyl or $R_2$ is hydrogen and $R_3$ is acetyl, haloacetyl or an oxycarbonyl group of the formula

$$R_4\text{-O-C(O)-}$$

wherein $R_4$ is $C_1$-$C_6$ alkyl, $C_2$-$C_6$ alkenyl, $C_3$-$C_7$ cycloalkyl, benzyl, nitrobenzyl, diphenylmethyl, or a phenyl group of the formula

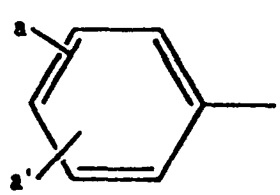

wherein a and a' independently are hydrogen, lower alkyl, lower alkoxy, halogen, trifluoromethyl, hydroxy, hydroxymethyl, amino, or aminomethyl; and the pharmaceutically acceptable non-toxic salts thereof.

**2.** A compound as claimed in claim 1, for use as an antithrombotic agent.

**3.** A pharmaceutical formulation comprising as an active ingredient a compound as claimed in claim 1, associated with one or more pharmaceutically acceptable carriers, excipients or diluents therefor.

**4.** The use of a compound according to claim 1, for the manufacture of a medicament for preventing the formation of clots.

**Claim for the following Contracting States : ES, GR**

**1.** A process for preparing a compound of the formula

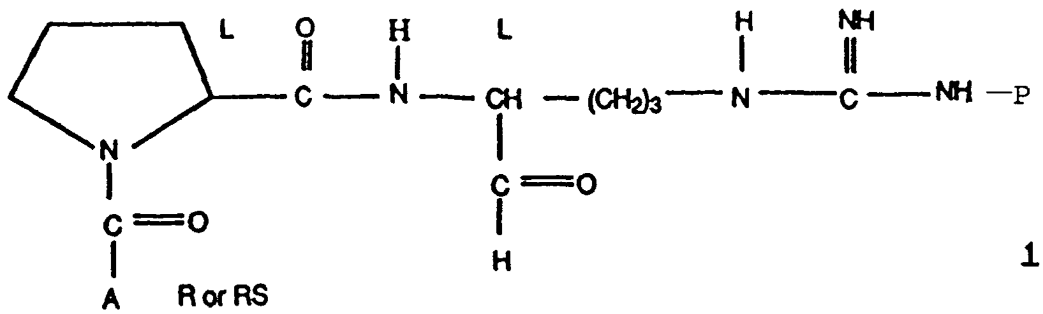

1

wherein A is 1-aminocyclohexyl or 1-aminocyclopentyl wherein the amino group is an $-N(R_2)(R_3)$ group wherein $R_2$ and $R_3$ independently are hydrogen or lower alkyl or $R_2$ is hydrogen and $R_3$ is acetyl, haloacetyl or an oxycarbonyl group of the formula

$$R_4\text{-O-C(O)-}$$

wherein $R_4$ is $C_1$-$C_6$ alkyl, $C_2$-$C_6$ alkenyl, $C_3$-$C_7$ cycloalkyl, benzyl, nitrobenzyl, diphenylmethyl, or a phenyl group of the formula

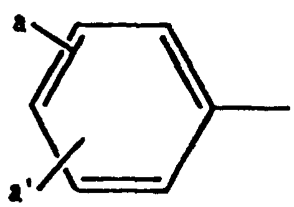

wherein a and a' independently are hydrogen, lower alkyl, lower alkoxy, halogen, trifluoromethyl, hydroxy, hydroxymethyl, amino, or aminoethyl; and P is hydrogen; and the pharmaceutically acceptable non-toxic salts thereof; which comprises removing the blocking group from a compound 1 wherein P is an amino protecting group.

## Patentansprüche

### Patentansprüche für folgende Vertragsstaaten : AT, BE, CH, LI, DE, DK, FR, GB, IT, LU, NL, SE

**1.** Verbindung der Formel

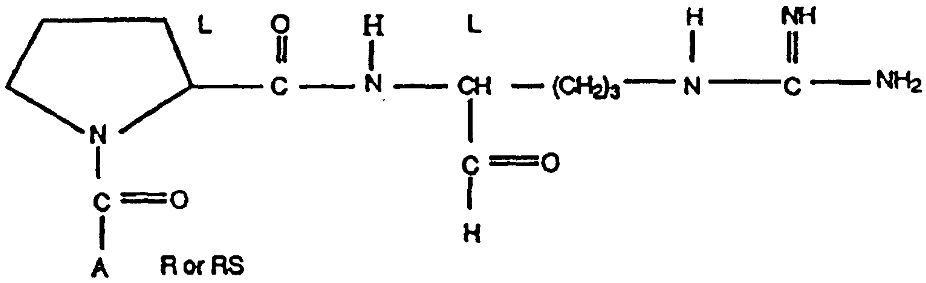

1

worin A 1-Aminocyclohexyl oder 1-Aminocyclopentyl ist, wobei die Aminogruppe -N($R_2$)($R_3$) ist, worin $R_2$ und $R_3$ unabhängig voneinander Wasserstoff oder ein Niederalkyl oder $R_2$ Wasserstoff und $R_3$ Acetyl, Halogenacetyl oder eine Oxycarbonylgruppe der Formel

$$R_4\text{-O-C(O)-}$$

ist, wobei $R_4$ $C_1$-$C_6$-Alkyl, $C_2$-$C_6$-Alklenyl, $C_3$-$C_7$-Cyclohexyl, Benzyl, Nitrobenzyl, Diphenylmethyl oder eine Phenylgruppe der Formel

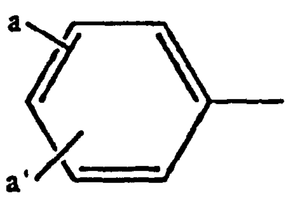

ist, worin a und a' unabhängig voneinander Wasserstoff, Niederalkyl, Niederalkoxy, Halogen, Trifluormethyl, Hydroxy, Hydroxymethyl, Amino oder Aminomethyl sind; und die pharmazeutisch verträglichen ungiftigen Salze davon.

**2.** Verbindung nach Anspruch 1 zur Verwendung als gerinnungshemmendes Mittel.

**3.** Pharmazeutische Formulierung, umfassend als einen wirksamen Bestandteil eine Verbindung nach Anspruch 1, zusammen mit einem oder mehreren pharmazeutisch verträglichen Trägern, Streckmitteln oder Verdünnern hierfür.

**4.** Verwendung einer Verbindung nach Anspruch 1 zur Herstellung eines Arzneimittels zur Verhinderung der Bildung von Blutgerinnsel.

### Patentanspruche für folgende Vertragsstaaten : ES, GR

**1.** Verfahren zur Herstellung einer Verbindung der Formel

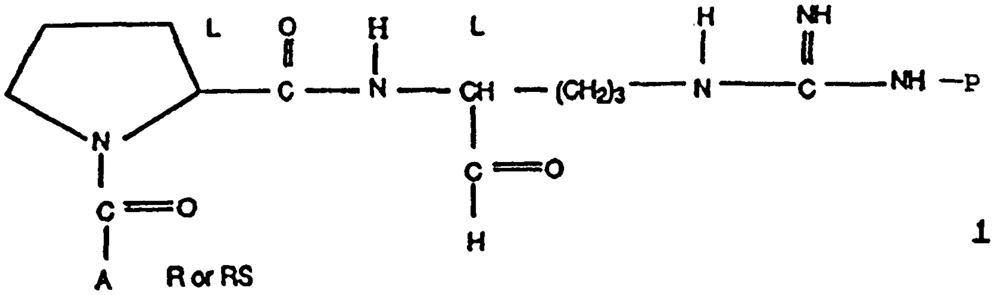

R or RS

1

worin A 1-Aminocyclohexyl oder 1-Aminocyclopentyl ist, wobei die Aminogruppe $-N(R_2)(R_3)$ ist, worin $R_2$ und $R_3$ unabhängig voneinander Wasserstoff oder Niederalkyl oder $R_2$ Wasserstoff und $R_3$ Acetyl, Halogenacetyl oder eine Oxycarbonylgruppe der Formel

$$R_4\text{-O-C(O)-}$$

ist, wobei $R_4$ $C_1$-$C_6$-Alkyl, $C_2$-$C_6$-Alkenyl, $C_3$-$C_7$-Cycloalkyl, Benzyl, Nitrobenzyl, Diphenylmethyl oder eine Phenylgruppe der Formel

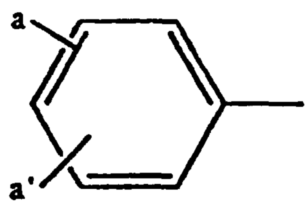

ist, worin a und a' unabhängig voneinander Wasserstoff, Niederalkyl, Niederalkoxy, Halogen, Trifluormethyl, Hydroxy, Hydroxymethyl, Amino, oder Aminomethyl sind; und P Wasserstoff ist; und die pharmazeutisch verträglichen ungiftigen Salze davon; welches die Entfernung der blockierenden Gruppe von Verbindung 1 umfasst, worin P eine Aminoschutzgruppe ist.

**Revendications**

**Revendications pour les Etats contractants suivants : AT, BE, CH, LI, DE, DK, FR, GB, IT, LU, NL, SE**

**1.** Composé répondant à la formule

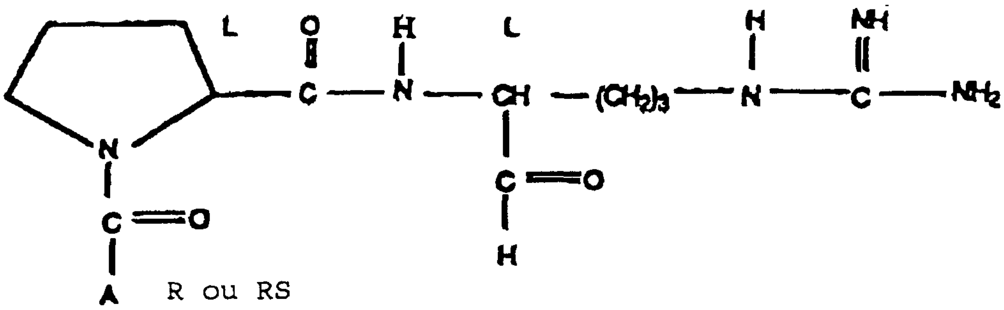

R ou RS

1

dans laquelle A représente un groupe 1-aminocyclohexyle ou un groupe 1-aminocyclopentyle dans lequel le groupe amino représente un groupe -N($R_2R_3$) dans lequel $R_2$ et $R_3$ représentent, indépendamment l'un de l'autre, un atome d'hydrogène ou un groupe alkyle inférieur ou bien $R_2$ représente un atome d'hydrogène et $R_3$ représente un groupe acétyle, un groupe halogénacétyle ou un groupe oxycarbonyle répondant à la formule

$$R_4\text{-O-C(O)-}$$

dans laquelle $R_4$ représente un groupe alkyle en $C_1$-$C_6$, un groupe alcényle en $C_2$-$C_6$, un groupe cycloalkyle en $C_3$-$C_7$, un groupe benzyle, un groupe nitrobenzyle, un groupe diphénylméthyle ou un groupe phényle répondant à la formule

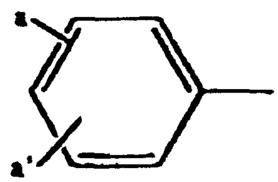

dans laquelle a et a' représentent, indépendamment l'un de l'autre, un atome d'hydrogène, un groupe alkyle inférieur, un groupe alcoxy inférieur, un atome d'halogène, un groupe trifluorométhyle, un groupe hydroxyle, un groupe hydroxyméthyle, un groupe amino ou un groupe aminométhyle; et ses sels non toxiques pharmaceutiquement acceptables.

2. Composé selon la revendication 1, à utiliser comme agent antithrombotique.

3. Formulation pharmaceutique comprenant, à titre d'ingrédient actif, un composé tel que revendiqué à la revendication 1, en association avec un ou plusieurs supports, excipients ou diluants pharmaceutiquement acceptables pour le composé.

4. Utilisation d'un composé selon la revendication 1, pour la préparation d'un médicament destiné à empêcher la formation de caillots.

**Revendication pour les Etats contractants suivants : ES, GR**

1. Procédé pour préparer un composé répondant à la formule

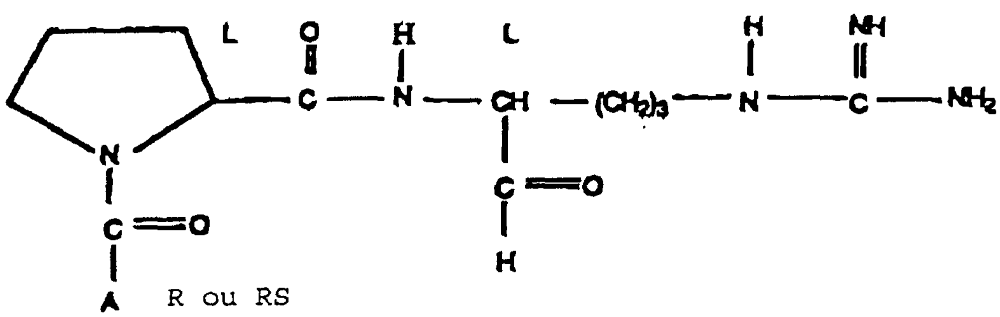

R ou RS

1

dans laquelle A représente un groupe 1-aminocyclohexyle ou un groupe 1-aminocyclopentyle dans lequel le groupe amino représente un groupe -N($R_2R_3$) dans lequel $R_2$ et $R_3$ représentent, indépendamment l'un de l'autre, un atome d'hydrogène ou un groupe alkyle inférieur ou bien $R_2$ représente un atome d'hydrogène et $R_3$ représente un groupe acétyle, un groupe halogénacétyle ou un groupe oxycarbonyle répondant à la formule

$R_4$-O-C(O)-

dans laquelle $R_4$ représente un groupe alkyle en $C_1$-$C_6$, un groupe alcényle en $C_2$-$C_6$, un groupe cycloalkyle en $C_3$-$C_7$, un groupe benzyle, un groupe nitrobenzyle, un groupe diphénylméthyle ou un groupe phényle répondant à la formule

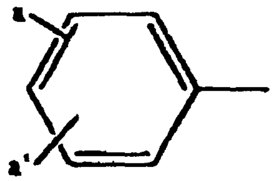

dans laquelle a et a' représentent, indépendamment l'un de l'autre, un atome d'hydrogène, un groupe alkyle inférieur, un groupe alcoxy inférieur, un atome d'halogène, un groupe trifluorométhyle, un groupe hydroxyle, un groupe hydroxyméthyle, un groupe amino ou un groupe aminoéthyle; et P représente un atome d'hydrogène; et ses sels non toxiques pharmaceutiquement acceptables; qui comprend le fait d'éliminer le groupe de blocage d'un composé répondant à la formule 1, dans lequel P représente un groupe protecteur du groupe amino.